# EUROPEAN PATENT SPECIFICATION

(11) **EP 2 708 190 B1**
(45) Date of publication and mention of the grant of the patent: **28.12.2016**
(21) Application number: 12782047.0
(22) Date of filing: 11.05.2012
(51) Int. Cl.: A61B 17/02, A61M 39/06, A61B 17/34, A61B 90/00

(54) **GUIDER PORT FOR ENDOSCOPIC SURGICAL INSTRUMENT, AND GAS-DISCHARGE VALVE FOR GUIDER PORT FOR SURGICAL INSTRUMENT**
FÜHRUNGSANSCHLUSS FÜR EIN ENDOSKOPISCHES CHIRURGISCHES INSTRUMENT UND GASENTLADUNGSVENTIL FÜR DEN FÜHRUNGSANSCHLUSS FÜR EIN ENDOSKOPISCHES CHIRURGISCHES INSTRUMENT
ORIFICE DE DISPOSITIF DE GUIDAGE POUR UN INSTRUMENT CHIRURGICAL ENDOSCOPIQUE, ET SOUPAPE D'ÉVACUATION DE GAZ POUR UN ORIFICE DE DISPOSITIF DE GUIDAGE POUR UN INSTRUMENT CHIRURGICAL

(30) Priority: 12.05.2011 KR 20110044307; 12.05.2011 KR 20110044308
(43) Date of publication of application: 19.03.2014
(73) Proprietor: Seo, O-Nam, Gyeonggi-do 420-020 (KR)
(72) Inventor: Seo, O-Nam, Gyeonggi-do 420-020 (KR)
(74) Representative: Zardi, Marco
(86) International application number: PCT/KR2012/003690
(87) International publication number: WO 2012/153998

(56) References cited:
- WO-A1-2010/104259
- WO-A2-2010/041900
- KR-B1- 100 975 030
- US-A1- 2008 312 496
- US-A1- 2009 036 745

## Description

### [Technical Field]

The present invention relates to an endoscopic surgical tool guider port installed in a surgical hole to guide various surgical tools into an abdominal cavity, and more particularly to an endoscopic surgical tool guider port by which an operation can be continuously performed while extractions are temporarily preserved in an extraction bag formed in a tube body, without requiring to directly open the guider port several times to extract the extractions generated during the surgery, enabling the endoscopic surgery to be performed more smoothly and promptly.

A gas exhaust valve for a surgical tool guider port can be used, and more particularly a gas exhaust valve for a surgical tool guider port that is installed in an exhaust tube of the surgical tool guider port to guide various surgical tools into an abdominal cavity during an endoscopic surgery to easily exhaust gas through a simple manipulation whenever needed during the surgery, thereby enabling the endoscopic surgery to be performed more smoothly.

### [Background Art]

In general, since a laparoscopic endoscopic surgery (also, referred to as 'minimum invasive operation') requires a small surgical hole (cutaway window) as compared with a traditional laparotomy, an operation scar is cosmetically well-treated and the pain due to the operation is not severe. Further, due to the rapid recovery speed, a period for hospital treatment is short and a patient can promptly return to the normal life. Thus, the laparoscopic endoscopic surgeries have been recently performed for almost all diseases except for some cancers.

The endoscopic surgery corresponds to a method of punching a small hole in the belly of a patient by using an insertion surgical device called a trocar, in which at least one trocar is inserted into the belly and various surgical devices such as a forceps, a cutting device, an internal organ extraction device, and an endoscopic camera are introduced to an operated portion in the belly through the trocar to perform various operations such as a gall bladder removal surgery, a gall bladder calculus removal surgery, an appendectomy, and a general surgery.

Meanwhile, in recent years, endoscopic surgeries in which a wound is rarely left are being performed through a portion of a navel without trying a plurality of trocars and cuttings as described above to reduce the wound left in the belly of the patient and recover the patient promptly. In general, if a hole for an operation is punched in the navel of a human body, since the wound is not easily exposed to the outside even after the wound is healed up and is not visually recognized as a wound, an endoscopic surgery through a navel is currently preferred.

A surgical tool guider port installed in a surgical hole, for safely introducing various surgical tools into an abdominal cavity is required for an endoscopic surgery through a single surgical hole.

Endoscopic surgical tool guider ports having various configurations are currently disclosed. In particular, the applicant developed various endoscopic surgical tool guider ports that can be conveniently and safely installed in a surgical hole and the guider ports were patented. (Korean Patent Nos. 10-915882, 10-1027561, and 10-1027546)

However, extractions generated during a surgical operation cannot be easily and efficiently processed by the conventional surgical tool guider ports including the patents of the applicant, and the operation may be delayed.
introduced through an endoscopic surgical tool guider port, the process is troublesome and it is difficult to secure an operation field of view so that an operation cannot be smoothly performed. Further, since the extractions should be extracted to the outside by opening an upper portion of an endoscopic surgical tool guider port (in the case of an open-topped surgical tool guider port) or releasing the endoscopic surgical tool guider port, the process is inconvenient and an overall operation time is considerably delayed.

Meanwhile, the conventional surgical tool guider port may have various configurations, and basically includes a plurality of tool introduction parts for introducing various surgical tools at an upper end of the surgical tool guider port, a gas injection part for injecting gas (for example, CO2) to expand an abdomen of a patient, and a gas exhaust part for exhausting toxic gas or smoke generated during a surgery.

In particular, the gas exhaust part is necessary because toxic gas or smoke is generated if flash or blood vessels are burned in an operation process, in which case the toxic gas or smoke is harmful to a human body unless it is promptly exhausted and a smooth operation cannot be performed as it is difficult to secure an operation field of view. Thus, it is general to provide a gas opening/closing valve in the gas exhaust part to exhaust toxic gas or smoke only when necessary while normally preventing leakage of gas.

However, since a gas opening/closing valve applied to the conventional surgical tool guider port has a structure driven through rotation of an operation lever, it is troublesome to open and close the valve. In particular, since toxic gas or smoke may be exhausted several times during a surgical process, an inconvenience of use hinders.a smooth operation or delay an operation time.

US 2008/312496 discloses an endoscopic surgical tool guider according to the prior art.

### [Disclosure]

### [Technical Problem]

The present invention has been made in an effort to solve the above-mentioned problems, and it is an object of the present invention to provide an endoscopic surgical tool guider port by which extractions generated in an operation process are contained in an extraction bag formed in a tube body to be conveniently processed so that an operation can be performed more promptly and smoothly without hindering an operation field of view or delaying an operation time.

### [Technical Solution]

In order to solve these problems, according to an aspect of the present invention, there is provided an endoscopic surgical tool guider port installed in a surgical hole of a patient during an endoscopic surgery to safely guide various surgical tools introduced into a tube body through a tool introduction part provided at an upper portion thereof into an abdominal cavity, wherein an extraction bag for containing and preserving extractions generated during the surgery therein is formed in the tube body to protrude to the outside.

The extraction bag may be formed such that a lower end thereof is inclined downwardly inward from an entrance of the extraction bag.

A plurality of independent storage chambers partitioned by partitions, for separating and preserving extractions may be formed within the extraction bag.

### [Advantageous Effects]

According to the endoscopic surgical tool guider port of the present invention, since extractions generated in an operation process can be directly contained and stored in an extraction bag formed in a tube body so that the operation can be continued without having to use a device such as a separate extraction extracting bag hindering an operation field of view and having to open or releae the guide port during the surgical process, the endoscopic surgery can be performed more promptly and smoothly.

Further, since various extractions can be separated and preserved according to demands such as types or purposes of the extractions, a utility of the guider port is improved and the guider port can be used very conveniently, and since the guider port can be manufactured without increasing manufacturing costs through a simple structure, increasing economical efficiency.

### [Description of Drawings]

FIG. 1 is a perspective view showing an endoscopic surgical tool guider port according to an embodiment of the present invention;
FIG. 2 is a sectional view showing an extraction bag according to an embodiment of the present invention;
FIG. 3 is a sectional view showing an extraction bag according to another embodiment of the present invention;
FIG. 4 is a perspective view showing a gas exhaust valve;
FIG. 5 is a perspective view showing the gas exhaust valve when an external force is applied thereto;
FIG. 6 is a sectional view of the gas exhaust valve of FIG. 4;
FIG. 7 is a sectional view of the state of FIG. 5;
FIG. 8 is a view exemplifying an endoscopic surgical tool guider port in which the gas exhaust valve is installed; and FIGS. 9 and 10 are views exemplifying operations of the gas exhaust valve .

### [Best Mode]

### [Mode for Invention]

Hereinafter, an endoscopic surgical tool guider port according to exemplary embodiments of the present invention will be described in detail with reference to the accompanying drawings.

The embodiments of the present invention are provided to fully describe the present invention to those skilled in the art, and it is noted that the shapes and sizes of elements may be exaggerated in the drawings to clearly describe the elements.

In a description of the embodiments, items, such as known functions and known configurations, which are obvious to those skilled in the art will not be described when they may make the technical features of the present invention obscure.

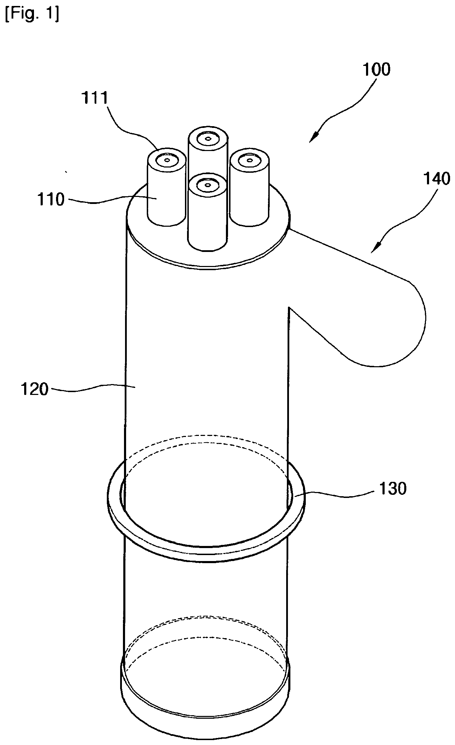

First, FIGS. 1 to 3 are views showing the endoscopic surgical tool guider port 100 according to an embodiment of the present invention. Referring to the drawings, the endoscopic surgical tool guider port 100 (hereinafter, referred to as "guider port") according to the embodiment of the present invention may include a tool entry part 110, a tube body 120, a support ring 130, an extraction bag 140 formed in the tube body 120.

The tool introduction part 110 is adapted to introduce various surgical tools such as a nipper and an endoscope for an operation into the tube body 120, and a valve unit 111 for easily introducing a surgical tool while maximally preventing leakage of gas is provided at an upper end of the tool introduction part 110. The tube body 120 has a hollow shape, and may be formed of a urethane sheet having excellent resiliency and durability.

The support ring 130 is an annular ring, and is caught by an abdominal wall in an abdominal cavity after passing through a surgical hole of a patient to support the guider port 100 when the guider port 100 is installed. Thus, the support ring 130 may be formed of a resilient material to be freely deformed such that the support ring 130 may easily pass the surgical hole and be folded or shrunk to be easily caught while being restored after the passing of the surgical hole, and to be restored to its original state immediately if an external force is removed after the deformation.

Meanwhile, various configurations such as the tool introduction part 110, the tube body 120, and the support ring 130 are already known in the art, and only one example is shown in the drawings.

Thus, it will be understood that the tool introduction part 110, the tube body 120, and the support ring 130 may be realized by various configurations and coupling relationships of known surgical tool guider ports without being limited to the configurations shown in the drawings.

Next, the extraction bag 140 is formed in the tube body 120, and has an interior space S for containing and preserving extractions generated in an operation process.

The extraction bag 140 protrudes from the tube body 120 outwards, so that an interior space of the tube body 120 is not influenced at all even if the extractions are preserved in the extraction bag 140. Thus, the extraction bas 140 does not hinder a field of view for an operation and movement of a surgical tool.

The extraction bag 140 may be integrally formed with the tube body 120, and thus may be formed of the same material as that of the tube body 120, that is, an urethane sheet having excellent resiliency and durability.

As shown in the drawings, the extraction bag 140 may be formed such that a lower end thereof is inclined downwardly inward from an entrance (a part connected to the tube body 120).

It can be seen that as a lower end of the extraction bag is downwardly inclined such that extractions contained and preserved in the extraction bag 140 can be prevented from being arbitrarily withdrawn from the extraction bag 140 during a surgical process so that the extractions can be easily preserved.

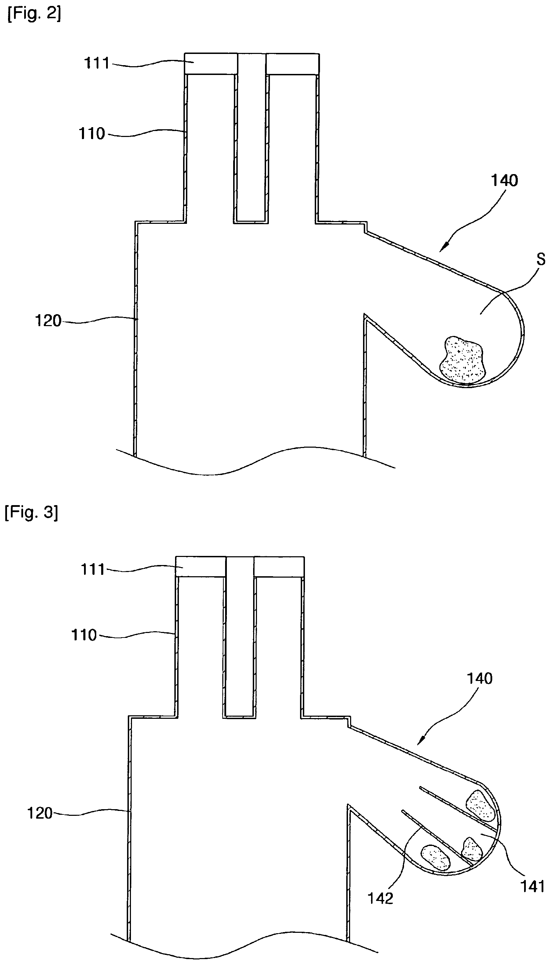

As shown in FIG. 3, a plurality of independent storage chambers 141 partitioned by partitions 142 may be formed in an interior of the extraction bag 140.

It can be seen that a plurality of extractions can be separated and preserved in the independent storage chambers 141 so as not to be mixed according to the types and purposes of the extractions.

The configuration of the surgical tool guider port 100 according to the embodiment of the present invention has been described until now. While the surgical tool guider port 100 according to the present invention is installed in a surgical hole of a patient, various surgical tools are introduced into an abdominal cavity along the tube body 120 through the tool introduction part 110 to perform an endoscopic surgery.

If various extractions such as tumors and gallstones are generated in the process of performing an endoscopic surgery, the extractions are directly contained in the extraction bag 140 formed in the tube body 120 so that the operation can be continuously performed instead of introducing a separate extraction extracting bag and containing and preserving the extractions or opening or releasing the surgical tool guider port 100, extracting the extractions to the outside, and reinstalling the surgical tool guider port 100 to perform the operation again

When the generated extractions need to be separated and preserved, an operation can be performed while the extractions are conveniently separated and preserved in the independent storage chambers 141 of the extraction bag 140.

It can be seen that if an endoscopic surgery is performed by using the surgical tool guider port 40 according to the present invention, extractions are preserved very conveniently so that the endoscopic surgery can be performed more smoothly without causing a delay in an operation time due to processing of the extractions at all.

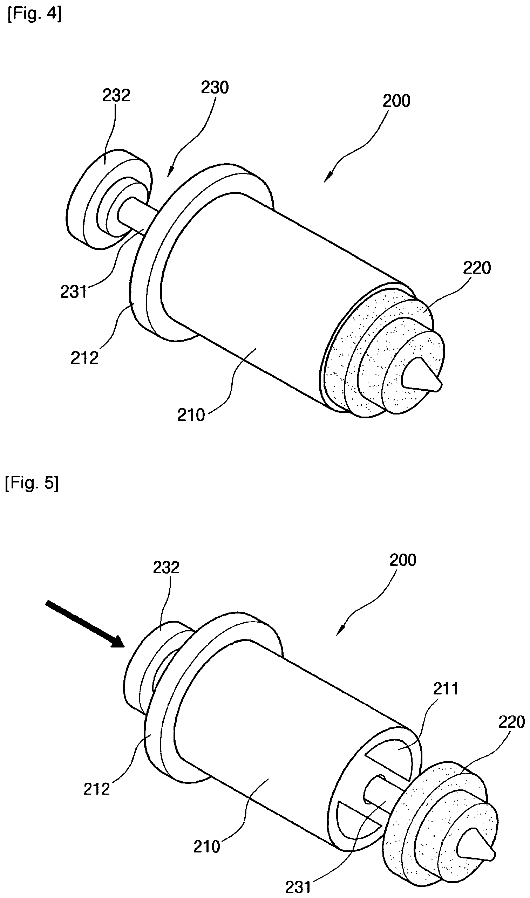

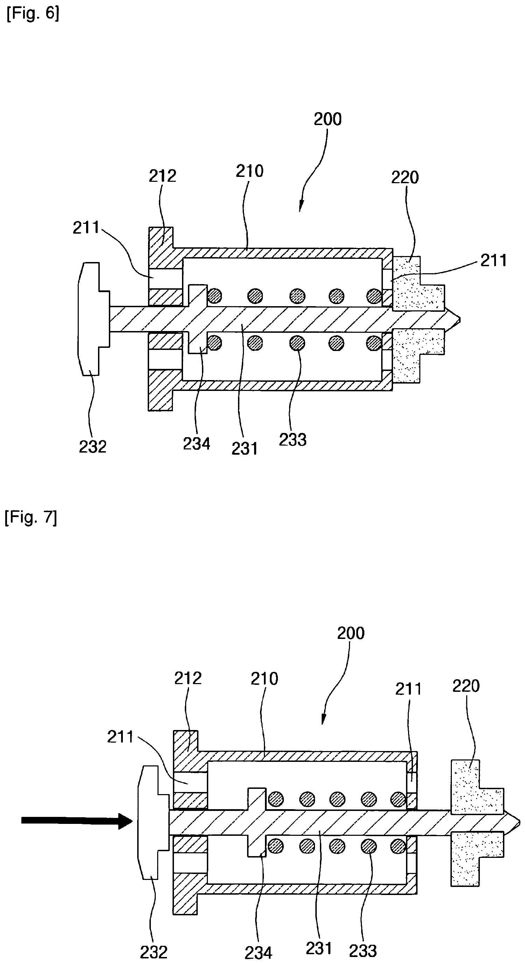

Meanwhile, FIGS. 4 to 7 are views showing the gas exhaust valve 200. As shown, the gas exhaust valve 200 may include a valve body 210, a packing member 220, and an actuating member 230.

The valve body 210 has a substantially cylindrical shape. A gas passage 211 is formed in the valve body 210 such that gas passes through the gas passage 211, and a body head 212 protrudes from an outer surface of one end of the valve body 210.

The packing member 220 is provided at one end of the valve body 210 to selectively open and dose the gas passage 211 of the valve body 210. That is, the packing member 220 blocks and closes the gas passage 211 while being attached to one end of the valve body 210 and opens the gas passage 211 while being separated from one end of the valve body 210.

The packing member 220 may be formed of a silicon material having a predetermined resiliency to show an excellent attaching force when the packing member 220 is attached to the valve body 210 to dose the gas passage 211.

If an external force is applied, the actuating member 230 separates the packing member 220 from the valve body 210 to open the gas passage 211, and if the external force is removed, the packing member 220 is attached to the valve body 210 to dose the gas passage 211.

The actuating member 230 may include a rod member 231, a button member 232, and a resilient member 233.

The rod member 231 passes through the valve body 210 to protrude from opposite sides of the valve body 210, and the packing member 220 is fixedly coupled to one of the protruding ends of the rod member 231. As the packing member 220 is fixedly coupled, the packing member 220 is moved in conjunction with the rod member 231.

The button member 232 is formed at a side of the rod member 231 opposite to the protruding end of the rod member 231, to which the packing member 220 is coupled, and the button member 232 is a part to which an external force is applied by the user, so that the connected rod member 231 is moved forward if an external force is applied.

The resilient member 233 is installed within the valve body 210 such that the rod member 231 is moved rearwards to an original position if the external force applied to the button member 232 is removed.

Thereto, the resilient member 233 should provide a resilient restoring force to the rod member 231, and the resilient member 233 may be realized by a spring that is fitted with the rod member 231 while being supported by a support 234 formed in the rod member 231.

The gas exhaust valve 200 is fixedly installed in an exhaust tube 300 provided at an upper end of the surgical tool guider port.

Here, the configuration of the surgical tool guider port can make the feature of the gas exhaust valve 200 excessively obscure, and thus a detailed description thereof will be omitted. It is noted that the gas exhaust valve 200 is not limited to be installed only in the surgical tool guider port having a specific configuration.

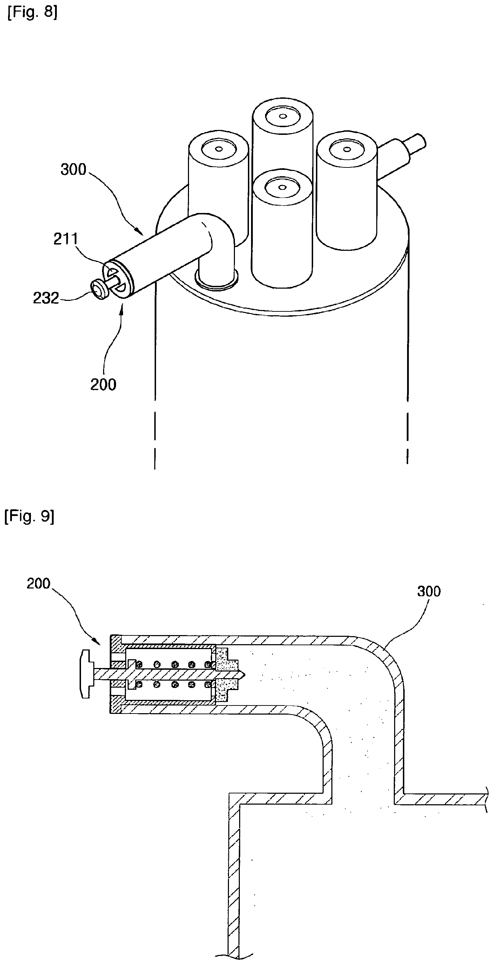

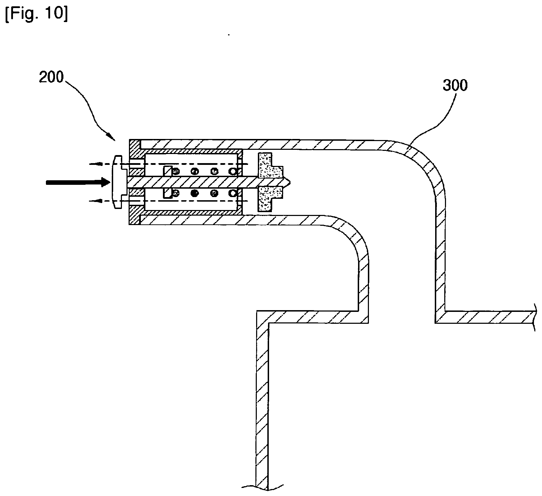

FIG. 8 exemplifies a state in which the gas exhaust valve 200 is installed in the exhaust tube 300 of the surgical tool guider port, and FIGS. 9 and 10 exemplify an operation of the gas exhaust valve 200. Hereinafter, an installation and an operation of the gas exhaust valve 200 will be described with reference to the drawings.

First, the gas exhaust valve 200 is installed in the exhaust tube 300, and the valve body 210 is inserted into the exhaust tube 300 to be installed. Then, the protruding body head 212 of the valve body 210 can be fixed to an end of the exhaust tube 300 more firmly while being attached to the end of the exhaust tube 300.

If the exhaust tube 300 is fixedly installed, the gas exhaust valve 200 is normally in a state in which the packing member 200 is attached to the valve body 210 and the gas passage 211 is dosed to stop leakage of gas.

When toxic gas or smoke is generated in an abdominal cavity or an interior of the surgical tool guider port through an operation of burning flash or vessels in the process of performing an operation through the surgical tool guider port

If the button member 232 is pushed to apply an external force, the rod member 231 is moved forward as shown in FIG. 10 while the button member 232 is pushed, and the packing member 220 coupled to the rod member 231 is moved in conjunction with the rod member 231 to be separated from the valve body 210 at the same time.

Thus, the gas passage 211 of the valve body 210 is opened, and toxic gas or smoke generated along the opened gas passage 211 is promptly exhausted.

If the exhaustion is completed, the applied external force has only to be removed by releasing the pushing of the button member 232.

The rod member 231 is moved rearwards while the compressed resilient member 233 provides a resilient restoring force to the rod member 231, and the packing member 220 doses the gas passage 211 while being attached to the valve body 210 again to stop leakage of gas.

It can be seen that the gas exhaust valve 200 can conveniently exhaust or block gas through a simple manipulation of a one touch button, and it is expected to contribute to a smoother endoscopic surgery through improvement of convenience of use.

Although the embodiments of the present invention have been described until now, the technical scope of the present invention is not limited by the embodiments and the contents of the drawings but is defined by the claims.

## Claims

1. An endoscopic surgical tool guider port (100) configured to be installed in a surgical hole of a patient during an endoscopic surgery to safely guide various surgical tools introduced into a tube body (120) through a tool introduction part (110) provided at an upper portion of the tool guider port into an abdominal cavity, the endoscopic surgical tool guider port (100) **characterized by** comprising:
an extraction bag (140) formed in the tube body (120) for containing and preserving extractions generated during the surgery therein, the extraction bag (140) protruding to an outside of the tube body.

2. The endoscopic surgical tool guider port (100) of claim 1, wherein the extraction bag (140) has a lower end inclined downwardly inward from an entrance of the extraction bag (140).

3. The endoscopic surgical tool guider port (100) of claim 1 or 2, wherein a plurality of independent storage chambers (141) partitioned by partitions (142) for separating and preserving extractions are formed within the extraction bag (140).

## Patentansprüche

1. Führungsanschluss (100) für ein endoskopisches chirurgisches Werkzeug, der so konfiguriert ist, dass er während eines endoskopischen Eingriffs in einer chirurgischen Öffnung eines Patienten installiert werden kann, um verschiedene chirurgische Werkzeuge, die durch ein an einem oberen Abschnitt des Werkzeugführungsanschlusses vorgesehenes Werkzeugeinführungsteil (110) in einen Tubus (120) eingeführt werden, sicher in eine Bauchhöhle zu führen, wobei der Führungsanschluss (100) für ein endoskopisches chirurgisches Werkzeug **dadurch gekennzeichnet ist, dass** er einen Extraktionsbeutel (140) umfasst, der in dem Tubus (120) ausgebildet ist, um Extraktate aufzunehmen und aufzubewahren, die während des Eingriffs darin erzeugt werden, wobei der Extraktionsbeutel (140) zu einer Außenseite des Tubus hin vorsteht.

2. Führungsanschluss (100) für ein endoskopisches chirurgisches Werkzeug nach Anspruch 1, wobei der Extraktionsbeutel (140) ein unteres Ende hat, das von einem Eingang des Extraktionsbeutels (140) nach innen und unten geneigt ist.

3. Führungsanschluss (100) für ein endoskopisches chirurgisches Werkzeug nach Anspruch 1 oder 2, wobei eine Vielzahl von unabhängigen Lagerungskammern (141), die durch Trennwände (142) unterteilt sind, zum Trennen und Aufbewahren von Extraktaten in dem Extraktionsbeutel (140) ausgebildet ist.

## Revendications

1. Port de guidage (100) d'un instrument chirurgical endoscopique destiné à être installé dans un orifice chirurgical d'un patient pendant une opération de chirurgie endoscopique pour un guidage sûr de différents instruments chirurgicaux introduits dans un corps tubulaire (120) à travers une pièce d'introduction (110) de l'instrument prévue au niveau d'une partie supérieure de l'orifice du dispositif de guidage de l'outil dans une cavité abdominale, le port de guidage (100) de l'instrument chirurgical endoscopique étant **caractérisé en ce qu'**il comprend :
un sac d'extraction (140) formé dans le corps tubulaire (120) pour contenir et préserver des extractions générées à l'intérieur pendant la chirurgie, le sac d'extraction (140) dépassant à l'extérieur du corps tubulaire.

2. Port de guidage (100) d'un instrument chirurgical selon la revendication 1, **caractérisé en ce que** le sac d'extraction (140) a une extrémité inférieure inclinée vers le bas vers l'intérieur à partir d'une entrée du sac d'extraction (140).

3. Port de guidage d'un outil chirurgical endoscopique (100) selon la revendication 1 ou 2, **caractérisé en ce qu'**une pluralité de chambres de stockage indépendantes (141) séparées par des partitions (142) afin de séparer et de préserver les extractions sont formées à l'intérieur du sac d'extraction (140).
